# EUROPEAN PATENT APPLICATION

(11) **EP 4 015 611 A1**
(43) Date of publication of application: **22.06.2022**
(21) Application number: 20215844.0
(22) Date of filing: 18.12.2020
(51) Int. Cl.: C11D 3/386, C12N 9/28

(54) **SHELF STABLE AMYLASE MUTANTS**

(71) Applicant: Clariant Produkte (Deutschland) GmbH, 65929 Frankfurt am Main (DE)
(72) Inventor: SCHATTE, Martin, 81476 München (DE); JOCHENS, Helge, 82065 Baierbrunn (DE); EISELE, Thomas, 77654 Offenburg (DE); STENZEL, Katharina, 82131 Gauting (DE); GLÜCK, Claudia, 70794 Filderstadt (DE); FRÜSTÜCK, Mona, 82152 Planegg (DE); LIST, Felix, 81476 München (DE); O'CONNELL, Timothy, 86899 Landsberg am Lech (DE); GRÄBER, Martin, 82131 Stockdorf (DE)
(74) Representative: Graser, Konstanze

(57) **Abstract**

The present invention relates to a novel shelf stable amylase mutant, the use of the novel amylase mutant in a laundry composition, a laundry composition comprising the novel amylase mutant and a method for laundering fabric.

## Description

The present invention relates to a novel shelf stable amylase mutant, the use of the novel amylase mutant in a laundry composition, a laundry composition comprising the novel amylase mutant and a method for laundering fabric.

As amylases are used for the initial stages (liquefaction) of starch amylases are of particular importance for many applications and are mandatory for the good performance of laundry applications. There are various amylases known within the art, however, functional requirements are constantly increasing as customers expect high cleaning performance while production costs and thus costs of the final product should be kept low. Amylases which can be flexibly used in different consumer products need to show a good activity over a wide temperature range and temperature stability at elevated temperatures is an important factor. However, it is often forgotten that temperature stability is also important during storage of the consumer product (e.g. the laundry composition) itself. Storage temperature is usually room temperature i.e. 20°C and therefore activity and washing performance may already decrease before the actual application of the product. Especially as the average storage time is 22 days.

The inventors of the present invention have therefore set themselves the task to develop a novel amylase suitable for use within laundry compositions, which shows significantly improved properties in view of shelf stability with regard to amylases known within the state of the art. It was a further task of the inventors of the present invention that the novel amylases are not only shelf stable but also maintain a good activity at a broad range of washing temperatures.

The inventors of the present invention have now surprisingly found that this task can be met by a shelf stable amylase mutant having a sequence identity of at least 90 % to SEQ ID NO: 2 and comprising a substitution at position 226.

Within a preferred embodiment, the inventive amylase mutant comprises comprising a sequence with at least 91%, preferably at least 92%, further preferred at least 94%, even more preferred at least 95%, also preferred at least 97%, particularly preferred at least 98% and most preferred at least 99% sequence identity to SEQ ID NO: 2.

The sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues X 100)/(Length of Alignment - Total Number of Gaps in Alignment)

Within the present invention the term "shelf stable" or "shelf stability" refers to an amylase enzyme which maintains at least 90 % of its activity when stored at a temperature of 20°C for a duration of 22 days in a liquid detergent formulation containing 5.3% linear C10C13-alkyl benzene sulfonic acid, 9.3 % lauryl diglycol ether sulfate sodium salt, 2.8 %stripped palm kernel fatty acid, 5.2% lauryl alcohol ethoxylate, 1.7% sodium citrate,1.0% nonionic polyester, 2.4% sodium hydroxide, 1.2% ethanol, 1% sodium tetraborate and 70,1 % destilled water (all percentages relate to vol-%).

Within the present invention these conditions are also referred to as "storage conditions".

Within the present invention the term "amylase" refers to any enzyme of class EC 3.2.1 and refers to alpha amylases (EC 3.2.1.1), beta amylases (EC 3.2.1.2) and gamma amylases (EC 3.2.1.3) whereas the amylase according to the invention is an alpha amylase originating from the gram positive microorganism *Bacillus amyloliquefaciens* (UniProt ID BOFYX9) and contains 659 amino acid residues.

Within a preferred embodiment, the substitution at position 226 is A226V.

The amino acids are thereby encoded as follows:
G Glycine, P Proline, A Alanine, V Valine, L Leucine, I Isoleucine, M Methionine, C Cysteine, F Phenylalanine, Y Tyrosine, W Tryptophan, H Histidine, K Lysine, R Arginine, Q Glutamine, N Asparagine, E Glutamic Acid, D Aspartic Acid, S Serine, T Threonine.

Within a further preferred embodiment of the present invention the shelf stable amylase mutant further contains at least one substitution at a position selected from the group consisting of positions 8, 294, 630, 121, 141, 172, 197, 571, 237, 432, 503, 124, 432, 229, 236, 474, 545, 143, 154 and 203. Within a preferred embodiment the at least one substitution at position 8, 294, 630, 121, 141, 172, 197, 571, 237, 432, 503, 124, 432, 229, 236, 474, 545, 143, 154 and/or 203 is N8D, S194G, H630P, A121S, N141D, R172S, N179D, M571I, D124N, N141D, D229N, M236L, K474R, G545S, S143F L154F, S203G and L154F.

Within a further preferred embodiment the shelf stable amylase mutant has sequence identity of at least 90 % to SEQ ID NO: 2 and comprises a substitution at position 226 as well as at least one further mutation at a position selected from the group consisting of 143, 154 and 203. Within a particularly preferred embodiment the shelf stable amylase mutant according to the present invention has sequence identity of at least 90 % to SEQ ID NO: 2 and comprises a substitution at position 226 as well as at least two further mutations at positions selected from the group consisting of 143, 154 and 203. Within most preferred embodiments the substitution(s) at position(s) 143, 154 and 203 are S143E, L154F and S203G.

Within another preferred embodiment, the shelf stable amylase mutant shows at least 50% of its activity when kept at a temperature selected from the range of from 20 to 50 °C, preferably selected from the range of from 20 to 40°C and most preferred at 40°C for a duration of 6 days. Storage conditions as defined before apply.

Within another preferred embodiment, the shelf stable amylase mutant shows at least 80% of its activity when kept at a temperature selected from the range of from 20 to 40 °C, preferably selected from the range of from 20 to 30°C and most preferred at 30°C for a duration of 15 days. Storage conditions as defined before apply.

Within a further preferred embodiment, the shelf stable amylase mutant shows at least 25% of its activity when kept at a temperature selected from the range of from 20 to 60 °C, preferably selected from the range of from 20 to 50°C and most preferred at 50°C for a duration of 1 day. Storage conditions as defined before apply.

Within another preferred embodiment the shelf stable amylase shows at least 80% activity when stored within a liquid detergent formulation as defined before at a pH selected from the range of from 7 to 9.5 for a duration of 20 days.

Within a further preferred embodiment the shelf stable amylase mutant shows a Lightness value L* of at least 25 when applied in a concentration of 0.025 mg/L. Within a preferred embodiment, the shelf stable amylase mutant shows a Lightness value L* of at least 35 when applied in a concentration of 0.05 mg/L and a Lightness value L* of at least 50 when applied in a concentration of 0.1 mg/L.

In another aspect the present invention relates to the use of the shelf stable amylase mutant for the production of a laundry composition. Any production method of a laundry composition as known to a person skilled in the art may be applied. The inventive laundry composition is preferably a liquid composition but may also be in granular or powder form.

In a further aspect the present invention also relates to a laundry composition (synonyms within the description: inventive composition, laundry composition, inventive laundry composition) containing the shelf stable amylase mutant and at least one further component selected from the group consisting further comprising at least one surfactant, builder, co-builder, anti-redeposition agent, dispersant, optical brightener, bleaching agent, dye, enzyme, chelator, polymer, pH buffering agent, filler, flocculant, fabric softener, foam booster, perfume, suds supressor, bacteriocide, fungicide, enzyme inhibitor, stabilizer, solubilizer, antioxidant, anti-corrosion agent and/or pigment.

The inventive laundry composition may include from 0.0001 to 10 weight-%, preferably from 0.0005 to 8 weight.-% such as from 0.001 to 5 weight-%, preferably of from 0.002 to 2 weight-%, particularly preferred of from 0.003 to 1 weight-% of the inventive shelf stable amylase mutant relative to the weight of the composition wherein ranges of from 0.0005 to 0.5 weight-%, for example from 0.0005 to 0.05 weight-% are also preferred.

Within the inventive laundry composition the shelf stable amylase mutant may be stabilized using conventional stabilizing agents for example a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, peptide aldehydes or ketones.

Within a preferred embodiment, the inventive composition comprises a shading dye. Preferably, the shading dye is a blue or violet shading dye, although other hues are also within the scope of the invention. The use of a blue or violet shading dye may enhance the perception of whiteness and / or cleanness.

Preferably, the shading dye is a reactive dye covalently bound to a polymer. More preferably, the polymer is a polyimine, optionally but preferably wherein the polyimine is substituted with 2-hydroxypropan-1-yl groups.

Within a preferred embodiment, the inventive composition comprises from 0.1 to 80 weight-%, preferably from 1 to 70 weight-%, more preferably from 2 to 60 weight-%, particularly preferred of from 4 to 40 weigh-%, even more preferred of from 5 to 35 weight-% and most preferred of from 6 to 33 weight-% of a surfactant.

Suitable surfactants may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981 or in Anionic Surfactants: Organic Chemistry edited by Helmut W. Stache (Marcel Dekker 1996).

Within a preferred embodiment, the inventive composition comprises at least one anionic surfactant preferably selected from water-soluble alkali metal salts of organic sulphates and sulphonates having alkyl radicals containing from about 8 to about 22 carbon atoms, the term alkyl being used to include the alkyl portion of higher alkyl radicals.

Examples of suitable synthetic anionic surfactants are sodium and potassium alkyl sulphates, especially those obtained by sulphating higher C₈ to C₁₈ alcohols, produced for example from tallow or coconut oil, alkyl ether carboxylic acids; sodium and potassium alkyl C₉ to C₂₀ benzene sulphonates, particularly sodium linear secondary alkyl C₁₀ to C₁₅ benzene sulphonates; and sodium alkyl glyceryl ether sulphates, especially those ethers of the higher alcohols derived from tallow or coconut oil and synthetic alcohols derived from petroleum and is preferably selected from linear alkyl benzene sulphonate; alkyl sulphates; alkyl ether sulphates; alkyl ether carboxylates; soaps; alkyl (preferably methyl) ester sulphonates, and mixtures thereof. Particularly preferred are alkyl ether sulphates such as C₁₂-C₁₄ n-alkyl ether sulphates with an average of 1 to 3 EO (ethoxylate) units. Sodium lauryl ether sulphate is particularly preferred (SLES). Preferably the linear alkyl benzene sulphonate is a sodium C₁₁ to C₁₅ alkyl benzene sulphonates. Preferably the alkyl sulphates is a linear or branched sodium C₁₂ to C₁₈ alkyl sulphates. Sodium dodecyl sulphate is particularly preferred (SDS, also known as primary alkyl sulphate).

Within a preferred embodiment two or more anionic surfactants are present within the inventive laundry composition, for example linear alkyl benzene sulphonate together with an alkyl ether sulphate.

Within a particularly preferred embodiment, the anionic surfactant compound is selected from linear alkyl benzene sulphonates; alkyl sulphates; alkyl ether sulphates; and mixtures thereof.

The inventive laundry composition may comprise anionic and/or non-ionic surfactants.

Within a preferred embodiment, the inventive laundry composition contains at least one nonionic surfactant, preferably selected from the reaction products of compounds having an aliphatic hydrophobic group and a reactive hydrogen atom, for example, aliphatic alcohols, acids or amides, especially ethylene oxide either alone or with propylene oxide. Specific nonionic surfactants are the condensation products of aliphatic C₈ to C₁₈ primary or secondary linear or branched alcohols with ethylene oxide.

Preferably the alkyl ethoxylated non-ionic surfactant is a C₈ to C₁₈ primary alcohol with an average ethoxylation of 7EO to 9EO units.

If a non-ionic surfactant is present, then most preferably the non-ionic surfactant is an alcohol ethoxylate, more preferably a C₁₀-C₁₈ alcohol ethoxylate having an average of 3-10 moles of ethylene oxide, most preferably a C₁₂-C₁₅ alcohol ethoxylate having an average of 5-9 moles of ethylene oxide.

Within a particularly preferred embodiment, the inventive laundry composition comprises a surfactant composition comprising of from 4 to 40 weight-%, more preferably from 5 to 35 weight-%, most preferably from 6 to 33 weight-% of a surfactant that comprises an anionic surfactant, preferably comprising linear alkyl benzene sulphonates and nonionic surfactant. In this preferable surfactant mixture, most preferably the weight fraction of nonionic surfactant to anionic surfactant is < 0.5, for example from 0.1 to < 0.5. This means that it is preferable that the level of anionic surfactant is greater than the level of nonionic surfactant in the detergent composition.

Within a preferred embodiment, the inventive laundry composition preferably comprises a perfume. Preferably the perfume comprises at least one note (compound) from: alpha-isomethyl ionone, benzyl salicylate; citronellol; coumarin; hexyl cinnamal; linalool; pentanoic acid, 2-methyl-, ethyl ester; octanal; benzyl acetate; 1,6-octadien-3-ol, 3,7-dimethyl-, 3-acetate; cyclohexanol, 2-(1,1-dimethylethyl)-, 1-acetate; delta-damascone; beta-ionone; verdyl acetate; dodecanal; hexyl cinnamic aldehyde; cyclopentadecanolide; benzeneacetic acid, 2-phenylethyl ester;amyl salicylate; beta-caryophyllene; ethyl undecylenate; geranyl anthranilate; alpha-irone; beta-phenyl ethyl benzoate; alpa-santalol; cedrol; cedryl acetate; cedry formate; cyclohexyl salicyate; gamma-dodecalactone; and beta phenylethyl phenyl acetate.

Within a preferred embodiment, the inventive laundry composition preferably comprises a fluorescent agent (optical brightener). Fluorescent agents are well known to a person skilled in the art and many such fluorescent agents are available commercially. Usually, these fluorescent agents are supplied and used in the form of their alkali metal salts, for example, the sodium salts.

Preferred classes of fluorescents are di-styryl biphenyl compounds, e.g. Tinopal^{™} CBS-X, Diamine stilbene di-sulphonic acid compounds, e.g. Tinopal DMS pure Xtra and Blankophor (Trade Mark) HRH, and Pyrazoline compounds, e.g. Blankophor SN.

Preferred fluorescents are: sodium 2 (4-styryl-3-sulphophenyl)-2H-napthol[1,2-d]triazole, disodium 4,4'-bis{[(4-anilino-6-(N methyl-N-2 hydroxyethyl) amino 1,3,5-triazin-2-yl)]amino}stilbene-2-2' disulophonate, disodium 4,4'-bis{[(4-anilino-6-morpholino-1,3,5-triazin-2-yl)]amino} stilbene-2-2' disulphonate, and disodium 4,4'-bis(2-sulphostyryl)biphenyl.

The total amount of the fluorescent agent or agents used in the inventive laundry composition is preferably of from 0.0001 to 0.5 weight-%, more preferably from 0.005 to 2 weight-%, most preferably from 0.05 to 0.25 weight-%.

Within a preferred embodiment, the inventive laundry composition preferably comprises at least one further enzyme. If present, then the level of each enzyme in the laundry composition of the invention is from 0.0001 weight-% to 0.1 weight-%, preferably of from 0.005 to 0.1 weight-%. Further enzymes are preferably selected from the group of proteases, cellulases, other amylases (amylases other than those specified in the invention as defined by the claims as filed), lipases, peroxidases/oxidases, pectate lyases, mannanases or mixtures thereof.

Within a particularly preferred embodiment, the at least one further enzyme is selected from proteases, cellulases, alpha-amylases and lipases.

Within a preferred embodiment, the inventive laundry composition preferably comprises at least one builder preferably selected from calcium sequestrant materials, precipitating materials, calcium ion-exchange materials and mixtures thereof.

Preferred calcium sequestrant builder materials include alkali metal polyphosphates, such as sodium tripolyphosphate and organic sequestrants, such as ethylene diamine tetra-acetic acid.

Preferred precipitating builder materials include sodium orthophosphate and sodium carbonate.

Preferred calcium ion-exchange builder materials include the various types of water-insoluble crystalline or amorphous aluminosilicates, of which zeolites are well known representatives, e.g. zeolite A, zeolite B (also known as zeolite P), zeolite C, zeolite X and zeolite Y.

Within a preferred embodiment, the inventive laundry composition preferably comprises from 0.001 to 65 weight-%, preferably of from 0.01 to 50 weight-%, further preferred of from 0.1 to 35 weight-% and most preferred of from 0.5 to 30 weight-% of a builder or complexing agent such as ethylenediaminetetraacetic acid, diethylenetriamine-penta-acetic acid, alkyl- or alkenyl succinic acid or nitrilotriacetic acid.

Preferably the inventive laundry composition is a non-phosphate built laundry detergent formulation, i.e., contains less than 1 weight-%, preferably less than 0.8 weight-%, further preferred less than 0.5 weight-% of phosphate wherein ranges of from 0.001 to 5 weight-% and from 0.001 to 1 weight-% are also preferred.

Within a preferred embodiment, the inventive laundry composition preferably comprises one or more polymers. Example polymers are polyethyleneimine, poly(vinylpyrrolidone), poly(vinylpyridine-N-oxide), poly(vinylimidazole), carboxymethylcellulose, poly(ethylene glycol), poly(vinyl alcohol), polycarboxylates such as polyacrylates, maleic/acrylic acid copolymers and lauryl methacrylate/acrylic acid copolymers. A preferred polymer is polyethyleneimine.

The present invention also pertains to a method of laundering fabric, the method comprising contacting the fabric with the inventive laundry composition as defined within the present application. Within a preferred embodiment the contacting of the fabric with the inventive laundry composition occurs at 60 °C or less, 50 °C or less, 40 °C or less, 30 °C or less, preferably at 25 °C or less and most preferred at 22 °C or less wherein a range of from 20 to 60°C is preferred and a range of from 20 to 40 °C is even more preferred.

### Generally preferred embodiments

In the following, generally preferred embodiments of the present invention are listed which do not limit the scope of the invention and/or scope of the claims in any respect. The generally preferred embodiments illustrate particularly suitable embodiments of the inventive shelf stable amylase mutant and the inventive laundry composition containing the inventive shelf stable amylase mutant.

### Generally preferred embodiment 1

Shelf stable amylase mutant having a sequence identity of at least 90 % to SEQ ID NO: 2 and comprising a substitution at position 226 which is A226V and at least one further substitution selected from the group consisting of N8D, S194G, H630P, A121S, N141D, R172S, N179D, M571I, D124N, N141D, D229N, M236L, K474R, G545S, S143F L154F, S203G and L154F.

### Generally preferred embodiment 2

Shelf stable amylase mutant as with a sequence identity of at least 90 % to SEQ ID NO: 2 and comprising a substitution at position 226 as well as at least one further substitution at a position selected from the group consisting of 143, 154 and 203.

### Generally preferred embodiment 3

Shelf stable amylase mutant as defined by generally preferred embodiment 2 wherein substitution(s) at position(s) 226, 143, 154 and 203 are A226V, S143E, L154F and S203G.

### Generally preferred embodiment 4

Shelf stable amylase mutant as defined by generally preferred embodiment 1, 2 or 3 showing at least 50% activity within a temperature range of from 20 to 50°C for a duration of 6 days.

### Generally preferred embodiment 5

Laundry composition comprising at least one shelf stable amylase mutant as defined by any of generally preferred embodiments 1, 2, 3 or 4 and at least one further component selected from the group consisting further comprising at least one surfactant, builder, co-builder, anti-redeposition agent, dispersant, optical brightener, bleaching agent, dye, enzyme, chelator, polymer, pH buffering agent, filler, flocculant, fabric softener, foam booster, perfume, suds supressor, bacteriocide, fungicide, enzyme inhibitor, stabilizer, solubilizer, antioxidant, anti-corrosion agent and/or pigment.

### Figures and examples

The present invention is described by the following figures and examples. It is thereby emphasized that the figures and examples do not limit the scope of the invention and claims but merely constitute further illustration of the invention, inventive purpose and benefits achieved by the inventive method.

### List of figures

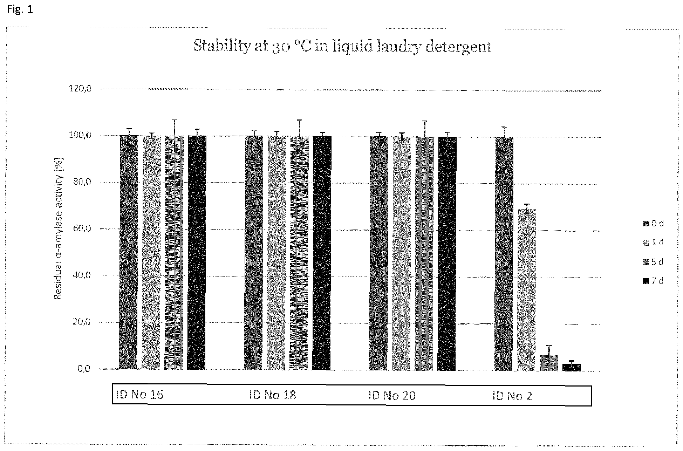

- Fig. 1:: Residual activity of selected shelf stable amylase mutants after storage at 30°C for indicated days.

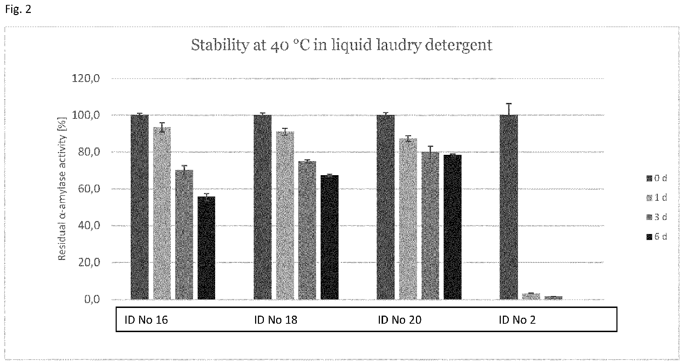

- Fig. 2:: Residual activity of selected shelf stable amylase mutants after storage at 40°C for indicated days.

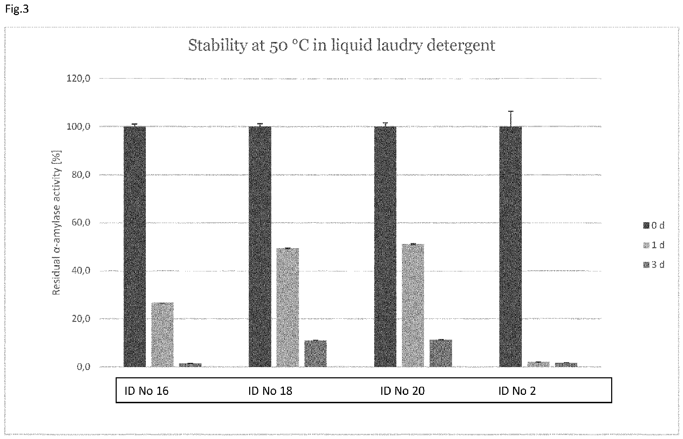

- Fig. 3:: Residual activity of selected shelf stable amylase mutants after storage at 50°C for indicated days.
- Fig. 4:: Wash-performance of selected shelf stable amylase mutants in different concentrations are shown. Soil removal was measured as the sum of delta L*-values of different soils (listed in experimental description)

### General

In the following section the generation of the shelf stable amylase mutants are descried.

**Table 1 shows the correlation between Seq ID No and the amino acids mutated in the shelf stable amylase mutants as well as the unmutated wild type**

| SEQ ID NO: | Mutation |
|---|---|
| 2 | Wild type |
| 4 | A226V |
| 6 | N8D, A226V, S294G |
| 8 | A226V, H630P |
| 10 | A121S, N141D, R172S, N179D, A226V, M5711 |
| 12 | A226V, N237S, A432T, T503I |
| 14 | D124N, N141D, A226V, D229N, M236L, K474R, G545S |
| 16 | S143E, L154F, A226V |
| 18 | L154F, S203G, A226V |
| 20 | S143E, S203G, A226V |

### Cloning and transformation of the wild type gene, which was used to generate the genes of shelf stable amylase mutants

Methods for manipulation of nucleic acid molecules are generally known to the skilled person in the field and are here introduced by reference (1. Molecular cloning: a laboratory manual, Michael R. Green and Joseph Sambrook, 4th edition; 2. Current Protocols in Molecular Biology, ISSN: 1934-3639).

SEQ ID NO: 1, coding for SEQ ID NO: 2 were synthesized by Geneart (Regensburg, Germany) and then used as template in PCR reactions for cloning in to pHT43 Vector SEQ ID NO: 21. Transformation was performed into competent *Bacillus subtilis (strain 168).*

### Introduction of mutations to generate the genes of shelf stable amylase mutants

The mutation of A226V was introduced, in to SEQ ID NO1, with side directed mutagenesis as described by (Kunkel, T. A. (1985) Proc Natl Acad Sci U S A 82(2):488-92) yielding SEQ ID No3. Therefore, an oligonucleotide with GTT, coding for V, flanged by 10 nucleotides to both sides which are matching the parent DNA was used. Residual parent DNA was removed as described by (Taylor, J. W., Ott, J. and Eckstein, F. (1985) Nucleic Acids Res 13(24):8765-85). All additional mutations (N8D, S194G, H630P, A121S, N141D, R172S, N179D, M571I, D124N, N141D, D229N, M236L, K474R, G545S, S143F L154F, S203G and L154F) were introduced iteratively using the same methods described above.

### Expression of shelf stable amylase mutants

The shelf stable amylase mutants were expressed in *Bacillus subtilis* (strain 168). A preculture of 50 ml Lysogeny Broth containing 100 µg/ml ampicillin was inoculated directly with the transformed *Bacillus subtilis* cells and grown over-night at 37 °C and 250 rpm shaking. Main cultures of 1500 ml Lysogeny Broth with 100 µg/ml ampicillin in 2L shake flasks were inoculated to an OD600 of 0.1 with the over-night culture and incubated at 37 °C and 250 rpm shaking. After 3 days supernatant was harvested by centrifugation (10 min, 10,000 x g).

### Example 1a: Stability of the shelf stable amylase mutants in a laundry composition at 20°C

For testing the stability of shelf stable amylase mutants in liquid laundry detergent, 200 µL of the appropriately diluted shelf stable amylase mutant were added to 3.8 g liquid detergent. The samples were mixed thoroughly using an overhead shaker and stored at the desired temperature. The activity of the shelf stable amylase mutants was measured before and after the storage at 20°C for 22 days. In detail, 0.2 g of the shelf stable amylase mutants containing detergent was weighed, diluted with 9.8 g assay buffer (100 mM maleate buffer, pH 6.5 with 1.54 mM CaCl₂) and stored on ice until measurement. For the measurement the samples were further diluted in assay buffer to ensure a measurement in the linear assay range. The "amylase HR assay reagent" (Megazyme u.c., Wicklow, Ireland) was used for activity determination. The "amylase HR assay reagent" contains p-nitrophenyl α-D-maltoheptaoside (blocked) as substrate and a thermostable α-glucosidase. The assay was conducted in 96-well plates at 37°C. After a 30 min incubation the reaction was stopped with Na₂CO₃ and the absorbance was measured at 400 nm using a microtiter plate photometer. Residual activity was calculated by division the absorbance at 400 nm at the respective time point by the absorbance at 400 nm before storage.

**Table 2: Residual activity of selected shelf stable amylase mutants after storage for 22 day at 20°C.**

| SEQ ID NO: | Mutation | Activity in % after 20°C for 22 Day |
|---|---|---|
| 2 | Wildtype, no mutation | 24 |
| 4 | A226V | 100 |
| 6 | N8D, A226V, S294G | 100 |
| 8 | A226V, H630P | 100 |
| 10 | A121S, N141D, R172S, N179D, A226V, M571I | 100 |
| 12 | A226V, N237S, A432T, T503I | 100 |
| 14 | D124N, N141D, A226V, D229N, M236L, K474R, G545S | 100 |
| 16 | S143E, L154F, A226V | 100 |
| 18 | L154F, S203G, A226V | 100 |
| 20 | S143E, S203G, A226V | 100 |

It can be seen from the results shown in table 1 that all tested mutants showed 100% of their activity after storage for 22 days at 20 °C while the respective wildtype enzyme lost 76%.

### Example 1b: Stability of the shelf stable amylase mutants in a laundry composition at 30°C

Conditions as described for example 1a but storage was carried out at 30°C for indicated days.

The results are shown in Fig,1

### Example 1c: Stability of the shelf stable amylase mutants in a laundry composition at 40°C

Conditions as described for example 1a but storage was carried out at 40°C for indicated days.

The results are shown in Fig,2

### Example 1d: Stability of the shelf stable amylase mutants in a laundry composition at 50°C

Conditions as described for example 1a but storage was carried out at 30°C for indicated days.

The results are shown in Fig,3

### Example 2: Launderometer wash test of shelf stable amylase mutants

Washing performance tests of selected shelf stable amylase mutants were performed in an Atlas LP-2 Launder-O-meter (SDS Atlas, Rock Hill, SC 29732, USA) as follows. The Launder-O-meter was first pre-heated to 40°C. The washing liquor was prepared using 4 g/L liquid laundry in synthetic tap water with defined German hardness of 14°, Ca:Mg 2:1. Subsequently, 32 g of steel balls (diameter 0.6 cm) and two swatches (5 x 4 cm) of each soiling were placed in the 1.2 L steel containers of the Launder-O-meter. Following soiled fabrics were used: C-S-26 corn starch colored on cotton, C-S-27 potato starch colored on cotton, C-S-28 rice starch colored on cotton, C-S-29 tapioca starch colored on cotton, C-S-126 corn starch colored aged at elevated temperature on cotton, C-S-127 potato starch colored aged at elevated temperature on cotton, C-S-128 rice starch colored aged at elevated temperature on cotton, C-S-129 tapioca starch colored aged at elevated temperature on cotton (Center for Testmaterials B.V. 3133 KT Vlaardingen, The Netherlands). Additionally, eight swatches of woven cotton fabric (10 x 8 cm, W-10A, Center for Testmaterials B.V. 3133 KT Vlaardingen, The Netherlands) were added in order to set the liquor cloth ratio to 25. Afterwards, 500 ml of wash liquor were added into the 1.2 L containers and the Launder-O-meter was run at 40°C for 30 min. Enzyme concentrations from of 0.1, 0.05 and 0.025 mg/L washing liquor were tested within the following laundry composition matrix:

**Table 2: laundry composition test matrix**

| **Component** | | | **Concentration** |
|---|---|---|---|
| | | Linear C10C13-alkyl benzene sulfonic acid | 5.3 % |
| | | Lauryl diglycol ether sulfate sodium salt | 9.3 % |
| | | Stripped palm kernel fatty acid | 2.8 % |
| | | Lauryl alcohol ethoxylate | 5.2 % |
| | | Sodium citrate | 1.7 % |
| | | Nonionic polyester | 1.0 % |
| | | Sodium hydroxide | 2.4 % |
| | | Ethanol | 1.2 % |
| | | Sodiumtetraborate | 1.0 % |

For each laundry composition a control sample without enzyme was accomplished. After the washing step in the Launder-O-meter, the swatches were rinsed with tap water by hand. The L*a*b* values of the soiled swatches were recorded after drying the textile over night at air using a spectrophotometer with D65, 10° (ColorFlex EZ, Hunterlab, Murnau am Staffelsee, Germany). The instrument was calibrated prior to the measurement with supplied white and black standards. Delta L*values (ΔL*) were calculated by subtracting the L* of the sample washed with enzyme from the L* of the sample washed without enzyme. Delta L*values of the different soilings were summed in order to compare the overall washing performance of the α-amylase variants.

The CIE L*a*b* color space is defined by the International Commission on Illumination and expresses color as three values: L* for the lightness from black (0) to white (100), a* from green (-) to red (+), and b* from blue (-) to yellow (+).

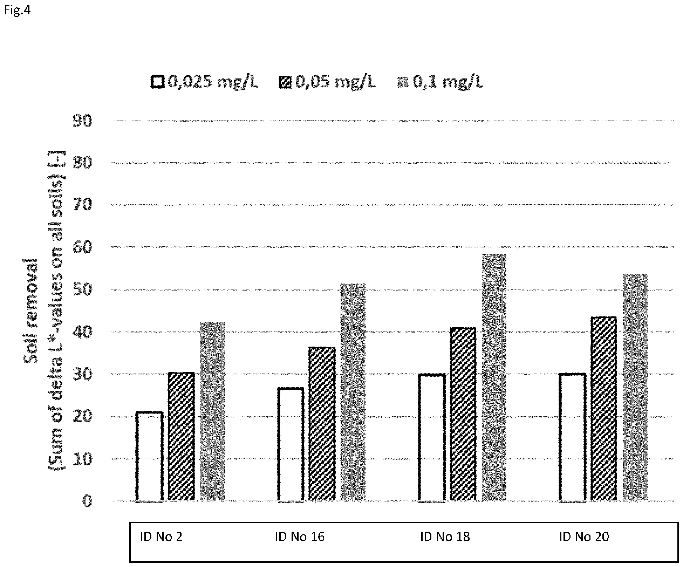

Results are shown in Figure 4.

## Claims

**1.** Shelf stable amylase mutant having a sequence identity of at least 90 % to SEQ ID NO: 2 and comprising a substitution at position 226.

**2.** Shelf stable amylase mutant according to claim 1, which further contains at least one substitution at a position selected from the group consisting of positions 8, 294, 630, 121, 141, 172, 197, 571, 237, 432, 503, 124, 432, 229, 236, 474, 545, 143, 154 and 203.

**3.** Shelf stable amylase mutant according to any of the foregoing claims, wherein the substitution at position 226 is A226V.

**4.** Shelf stable amylase mutant according to any of claims 2 to 3, wherein the at least one substitution at position 8, 294, 630, 121, 141, 172, 197, 571, 237, 432, 503, 124, 432, 229, 236, 474, 545, 143, 154 and/or 203 is N8D, S194G, H630P, A121S, N141D, R172S, N179D, M571I, D124N, N141D, D229N, M236L, K474R, G545S, S143F L154F, S203G and L154F.

**4.** Shelf stable amylase mutant according to any of the foregoing claims, showing a at least 50% activity within a temperature range of from 20 to 50 °C for a duration of 6 days.

**5.** Shelf stable amylase mutant according to any of the foregoing claims, showing a at least 80% activity within a temperature range of from 20 to 40 °C for a duration of 15 days.

**6.** Shelf stable amylase mutant according to any of the foregoing claims, showing a at least 25% activity within a temperature range of from 20 to 60 °C for a duration of 1 day.

**7.** Shelf stable amylase mutant according to any of the foregoing claims, showing at least 90% activity at a pH of from 7 to 9.5 for a duration of 20 days at 20 °C.

**8.** Shelf stable amylase mutant according to any of the foregoing claims, showing a Lightness value of at least 25 when applied in a concentration of 0.025 mg/L.

**9.** Use of the shelf stable amylase mutant according to any of claims 1 to 8, for the production of a laundry composition.

**10.** Laundry composition comprising the shelf stable amylase mutant according to any of claims 1 to 8 and at least one further component selected from the group consisting further comprising at least one surfactant, builder, co-builder, anti-redeposition agent, dispersant, optical brightener, bleaching agent, dye, enzyme, chelator, polymer, pH buffering agent, filler, flocculant, fabric softener, foam booster, perfume, suds supressor, bacteriocide, fungicide, enzyme inhibitor, stabilizer, solubilizer, antioxidant, anti-corrosion agent and/or pigment.

**11.** Method for laundering fabric comprising the step: contacting the fabric with water and an amount of from 1 to 10 vol.-% of the laundry composition according to claim 10.

**12.** Method according to claim 11, wherein the contacting is carried out at a temperature of from 20 to 60 °C.


SEQUENCE LISTING

<110> Clariant Produkte (Deutschland) GmbH

<120> Shelf stable amylase mutants

<130> 2020DE629-EP-EPA

<160> 21

<170> BiSSAP 1.3.6

<210> 1
<211> 1896
<212> DNA
<213> Bacillus amyloliquefaciens

<220>
<223> native gene

<400> 1
gctgcaaacg ctgaaactgc aaacaaatcg aatgaggtgg ccgcgtcatc ggtcaaaaac 60

gggaccatcc ttcatgcctg gaattggtcg ttcaatacgt taacacaaaa tatgaaagag 120

attcgtgatg cgggctatgc agccattcag acgtctccga ttaaccaagt aaaggaaggg 180

aaccaaggag ataaaagcat gaggaactgg tactggctgt atcagccgac atcgtaccaa 240

atcggcaacc gttacttagg cactgaacaa gaatttaagg acatgtgtgc agccgcggaa 300

aaatatggcg taaaagtcat tgttgatgcg gttatcaatc ataccaccag cgattacgcc 360

gcgatttctg acgagattaa gcgtattcca aactggaccc atggaaacac acaaattaaa 420

aattggtcgg acaggtggga cgtcactcaa aattcattgc ttgggctgta tgattggaat 480

acacagaata ctgaggtgca aacctatctg aaacgtttct tggaaagagc attgaatgac 540

ggagcggacg ggttccgcta tgatgccgcc aagcatatag agcttccgga tgatgggaat 600

tacggcagcc gattttggcc gaatatcaca aatacatcgg cggagttcca atacggagaa 660

atcctgcaag acagcgcgtc cagagatact gcttatgcga attatatgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga     1320

aatgcgcctc aggtctttct tgagaattac caaacggggg cagtccattc tttcaatgat     1380

cagctgacgg tcaccctgcg tgcaaatgcg aaaacaacaa aagccgttta ccaaatcaat     1440

aatgggcagc agacagcatt taaggatgga gaccgattaa cgatcgggaa aggagatcca     1500

atcggcacga catacaacat caggttaacc ggaacgaacg gcgagggtgc agagagaact     1560

caagaataca cgtttgtcaa aaaagacccg gcccaaacca acatcattgg ctatcaaaat     1620

ccggatcatt ggggccaggt aaatgcttat atttacaaac atgatggagg cagggccata     1680

gaattaaccg gatcgtggcc ggggaaagca atgactaaga atgcaaatgg aatttacacg     1740

ctgacgctgc ctgcgaatgc ggatacggcc aatgccaaag tgatttttaa caatggcagc     1800

gcccaagtgc ccggacagaa ccagcccggc tttgattatg tgcagaatgg tttgtataac     1860

aactccggtt tagacggtta tcttccgcat taataa                               1896

<210> 2
<211> 630
<212> PRT
<213> Bacillus amyloliquefaciens

<220>
<223> amylase

<400> 2
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Ala Ser Arg Asp Thr Ala Tyr Ala Asn Tyr Met Asn Val Thr Ala

225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 3
<211> 1896
<212> DNA
<213> Artificial Sequence

<220>
<223> mutant amylase

<400> 3
gctgcaaacg ctgaaactgc aaacaaatcg aatgaggtgg ccgcgtcatc ggtcaaaaac        60

gggaccatcc ttcatgcctg gaattggtcg ttcaatacgt taacacaaaa tatgaaagag 120

attcgtgatg cgggctatgc agccattcag acgtctccga ttaaccaagt aaaggaaggg 180

aaccaaggag ataaaagcat gaggaactgg tactggctgt atcagccgac atcgtaccaa 240

atcggcaacc gttacttagg cactgaacaa gaatttaagg acatgtgtgc agccgcggaa 300

aaatatggcg taaaagtcat tgttgatgcg gttatcaatc ataccaccag cgattacgcc 360

gcgatttctg acgagattaa gcgtattcca aactggaccc atggaaacac acaaattaaa 420

aattggtcgg acaggtggga cgtcactcaa aattcattgc ttgggctgta tgattggaat 480

acacagaata ctgaggtgca aacctatctg aaacgtttct tggaaagagc attgaatgac 540

ggagcggacg ggttccgcta tgatgccgcc aagcatatag agcttccgga tgatgggaat 600

tacggcagcc gattttggcc gaatatcaca aatacatcgg cggagttcca atacggagaa 660

atcctgcaag acagcgtttc cagagatact gcttatgcga attatatgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 4
<211> 630
<212> PRT
<213> Artificial Sequence

<220>
<223> mutant amylase

<400> 4
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415

Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
                580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 5
<211> 1896
<212> DNA
<213> Artificial Sequence

<220>
<223> mutant amylase

<400> 5
gctgcaaacg ctgaaactgc agataaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa     300

aaatatggcg taaaagtcat tgttgatgcg gttatcaatc ataccaccag cgattacgcc     360

gcgatttctg acgagattaa gcgtattcca aactggaccc atggaaacac acaaattaaa     420

aattggtcgg acaggtggga cgtcactcaa aattcattgc ttgggctgta tgattggaat     480

acacagaata ctgaggtgca aacctatctg aaacgtttct tggaaagagc attgaatgac     540

ggagcggacg ggttccgcta tgatgccgcc aagcatatag agcttccgga tgatgggaat     600

tacggcagcc gattttggcc gaatatcaca aatacatcgg cggagttcca atacggagaa     660

atcctgcaag acagcgtttc cagagatact gcttatgcga attatatgaa tgtgacggct     720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatgg gcgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 6
<211> 630
<212> PRT
<213> Artificial Sequence

<220>
<223> mutant amylase

<400> 6
Ala Ala Asn Ala Glu Thr Ala Asp Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95

Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Gly Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln

595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 7
<211> 1896
<212> DNA
<213> Artificial Sequence

<220>
<223> mutant amylase

<400> 7
gctgcaaacg ctgaaactgc aaacaaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa     300

aaatatggcg taaaagtcat tgttgatgcg gttatcaatc ataccaccag cgattacgcc     360

gcgatttctg acgagattaa gcgtattcca aactggaccc atggaaacac acaaattaaa     420

aattggtcgg acaggtggga cgtcactcaa aattcattgc ttgggctgta tgattggaat     480

acacagaata ctgaggtgca aacctatctg aaacgtttct tggaaagagc attgaatgac     540

ggagcggacg ggttccgcta tgatgccgcc aagcatatag agcttccgga tgatgggaat     600

tacggcagcc gattttggcc gaatatcaca aatacatcgg cggagttcca atacggagaa     660

atcctgcaag acagcgtttc cagagatact gcttatgcga attatatgaa tgtgacggct     720

tctaactatg ggcattccat cagatccgct ttaaagaacc gtaatctgag tgtgtcgaat     780

atctcccatt atgcatctga cgtgtctgcg gacaagttag tcacatgggt ggaatcacat     840

gatacgtatg ccaatgatga ggaagagtcc acatggatga gtgatgacga tatccgttta     900

ggctgggcag tgattggttc ccgctcagga agcacgcctc ttttcttttc cagacctgag     960

ggcggaggaa atggggtgcg atttcccgga aaaagtcaaa taggagatcg agggagcgcc    1020

ttatttaaag atcaggcgat cactgcggtc aatcaatttc acaatgtaat ggccgggcag    1080

cctgaggaac tctcgaatcc gaatgggaac aatcaaatat tgatgaatca gcgcggctca    1140

aaaggcgttg tgctggctaa tgcgggatcg tcttctgtca ccgtcaatac ttcaacgaaa    1200

ttgcctgacg gcaggtatga taatagggcc ggcgccggtt catttcaagt agcgaacggc    1260

aaactgacag gtacgatcaa tgccagatcc gcggctgttc tttattctga tgatattgga    1320

aatgcgcctc aggtctttct tgagaattac caaacggggg cagtccattc tttcaatgat    1380

cagctgacgg tcaccctgcg tgcaaatgcg aaaacaacaa aagccgttta ccaaatcaat    1440

aatgggcagc agacagcatt taaggatgga gaccgattaa cgatcgggaa aggagatcca    1500

atcggcacga catacaacat caggttaacc ggaacgaacg gcgagggtgc agagagaact    1560

caagaataca cgtttgtcaa aaaagacccg gcccaaacca acatcattgg ctatcaaaat    1620

ccggatcatt ggggccaggt aaatgcttat atttacaaac atgatggagg cagggccata    1680

gaattaaccg gatcgtggcc ggggaaagca atgactaaga atgcaaatgg aatttacacg    1740

ctgacgctgc ctgcgaatgc ggatacggcc aatgccaaag tgatttttaa caatggcagc    1800

gcccaagtgc ccggacagaa ccagcccggc tttgattatg tgcagaatgg tttgtataac    1860

aactccggtt tagacggtta tcttccgccg taataa                              1896

<210> 8
<211> 630
<212> PRT
<213> Artificial Sequence

<220>
<223> mutant amylase

<400> 8
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu

                275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro Pro
625                 630

<210> 9
<211> 1896
<212> DNA
<213> Artificial Sequence

<220>
<223> mutant amylase

<400> 9
gctgcaaacg ctgaaactgc aaacaaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa 300

aaatatggcg taaaagtcat tgttgatgcg gttatcaatc ataccaccag cgattacgcc 360

gttatttctg acgagattaa gcgtattcca aactggaccc atggaaacac acaaattaaa 420

gattggtcgg acaggtggga cgtcactcaa aattcattgc ttgggctgta tgattggaat 480

acacagaata ctgaggtgca aacctatctg aaaagcttct tggaaagagc attggatgac 540

ggagcggacg ggttccgcta tgatgccgcc aagcatatag agcttccgga tgatgggaat 600

tacggcagcc gattttggcc gaatatcaca aatacatcgg cggagttcca atacggagaa 660

atcctgcaag acagcgtttc cagagatact gcttatgcga attatatgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca attactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 10
<211> 630
<212> PRT
<213> Artificial Sequence

<220>
<223> mutant amylase

<400> 10
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Val Ile Ser Asp Glu Ile Lys Arg
        115                 120                 125
Ile Pro Asn Trp Thr His Gly Asn Thr Gln Ile Lys Asp Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Ser Phe Leu Glu Arg
                165                 170                 175
Ala Leu Asp Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460

Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Ile Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 11
<211> 1896
<212> DNA
<213> Artificial Sequence

<220>
<223> mutant amylase

<400> 11
gctgcaaacg ctgaaactgc aaacaaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa     300

aaatatggcg taaaagtcat tgttgatgcg gttatcaatc ataccaccag cgattacgcc     360

gcgatttctg acgagattaa gcgtattcca aactggaccc atggaaacac acaaattaaa     420

aattggtcgg acaggtggga cgtcactcaa aattcattgc ttgggctgta tgattggaat     480

acacagaata ctgaggtgca aacctatctg aaacgtttct tggaaagagc attgaatgac     540

ggagcggacg ggttccgcta tgatgccgcc aagcatatag agcttccgga tgatgggaat     600

tacggcagcc gattttggcc gaatatcaca aatacatcgg cggagttcca atacggagaa     660

atcctgcaag acagcgtttc cagagatact gcttatgcga attatatggt tgtgacggct     720

tctaactatg ggcattccat cagatccgct ttaaagaacc gtaatctgag tgtgtcgaat     780

atctcccatt atgcatctga cgtgtctgcg gacaagttag tcacatgggt ggaatcacat     840

gatacgtatg ccaatgatga ggaagagtcc acatggatga gtgatgacga tatccgttta     900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggatgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcagca catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 12
<211> 630
<212> PRT
<213> Artificial Sequence

<220>
<223> mutant amylase

<400> 12
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140

Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Val Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Asp
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Ser Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 13
<211> 1896
<212> DNA
<213> Artificial Sequence

<220>
<223> mutant amylase

<400> 13
gctgcaaacg ctgaaactgc aaacaaatcg aatgaggtgg ccgcgtcatc ggtcaaaaac 60

gggaccatcc ttcatgcctg gaattggtcg ttcaatacgt taacacaaaa tatgaaagag 120

attcgtgatg cgggctatgc agccattcag acgtctccga ttaaccaagt aaaggaaggg 180

aaccaaggag ataaaagcat gaggaactgg tactggctgt atcagccgac atcgtaccaa 240

atcggcaacc gttacttagg cactgaacaa gaatttaagg acatgtgtgc agccgcggaa 300

aaatatggcg taaaagtcat tgttgatgcg gttatcaatc ataccaccag cgattacgcc 360

gcgatttcta atgagattaa gcgtattcca aactggaccc atggaaacac acaaattaaa 420

gattggtcgg acaggtggga cgtcactcaa aattcattgc ttgggctgta tgattggaat 480

acacagaata ctgaggtgca aacctatctg aaacgtttct tggaaagagc attgaatgac 540

ggagcggacg ggttccgcta tgatgccgcc aagcatatag agcttccgga tgatgggaat 600

tacggcagcc gattttggcc gaatatcaca aatacatcgg cggagttcca atacggagaa 660

atcctgcaag acagcgtttc cagaaatact gcttatgcga attatctgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa gagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggagccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 14
<211> 630
<212> PRT
<213> Artificial Sequence

<220>
<223> mutant amylase

<400> 14
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asn Glu Ile Lys Arg
        115                 120                 125
Ile Pro Asn Trp Thr His Gly Asn Thr Gln Ile Lys Asp Trp Ser Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asn Thr Ala Tyr Ala Asn Tyr Leu Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp

325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Arg Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Ser Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 15
<211> 1896
<212> DNA
<213> Artificial Sequence

<220>
<223> mutant amylase

<400> 15
gctgcaaacg ctgaaactgc aaacaaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa     300

aaatatggcg taaaagtcat tgttgatgcg gttatcaatc ataccaccag cgattacgcc     360

gcgatttctg acgagattaa gcgtattcca aactggaccc atggaaacac acaaattaaa     420

aattgggaag acaggtggga cgtcactcaa aattcattgt ttgggctgta tgattggaat 480

acacagaata ctgaggtgca aacctatctg aaacgtttct tggaaagagc attgaatgac 540

ggagcggacg ggttccgcta tgatgccgcc aagcatatag agcttccgga tgatgggaat 600

tacggcagcc gattttggcc gaatatcaca aatacatcgg cggagttcca atacggagaa 660

atcctgcaag acagcgtttc cagagatact gcttatgcga attatatgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 16
<211> 630
<212> PRT
<213> Artificial Sequence

<220>
<223> mutant amylase

<400> 16
Ala Ala Asn Ala Glu Thr Ala Asn Lys Ser Asn Glu Val Ala Ala Ser

1               5                   10                  15
Ser Val Lys Asn Gly Thr Ile Leu His Ala Trp Asn Trp Ser Phe Asn
                20                  25                  30
Thr Leu Thr Gln Asn Met Lys Glu Ile Arg Asp Ala Gly Tyr Ala Ala
            35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Glu Asp
    130                 135                 140
Arg Trp Asp Val Thr Gln Asn Ser Leu Phe Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Ser Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510

Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 17
<211> 1896
<212> DNA
<213> Artificial Sequence

<220>
<223> mutant amylase

<400> 17
gctgcaaacg ctgaaactgc aaacaaatcg aatgaggtgg ccgcgtcatc ggtcaaaaac      60

gggaccatcc ttcatgcctg gaattggtcg ttcaatacgt taacacaaaa tatgaaagag     120

attcgtgatg cgggctatgc agccattcag acgtctccga ttaaccaagt aaaggaaggg     180

aaccaaggag ataaaagcat gaggaactgg tactggctgt atcagccgac atcgtaccaa     240

atcggcaacc gttacttagg cactgaacaa gaatttaagg acatgtgtgc agccgcggaa     300

aaatatggcg taaaagtcat tgttgatgcg gttatcaatc ataccaccag cgattacgcc     360

gcgatttctg acgagattaa gcgtattcca aactggaccc atggaaacac acaaattaaa     420

aattggtcgg acaggtggga cgtcactcaa aattcattgg aagggctgta tgattggaat     480

acacagaata ctgaggtgca aacctatctg aaacgtttct tggaaagagc attgaatgac     540

ggagcggacg ggttccgcta tgatgccgcc aagcatatag agcttccgga tgatgggaat     600

tacggctttc gattttggcc gaatatcaca aatacatcgg cggagttcca atacggagaa     660

atcctgcaag acagcgtttc cagagatact gcttatgcga attatatgaa tgtgacggct     720

tctaactatg ggcattccat cagatccgct ttaaagaacc gtaatctgag tgtgtcgaat     780

atctcccatt atgcatctga cgtgtctgcg gacaagttag tcacatgggt ggaatcacat     840

gatacgtatg ccaatgatga ggaagagtcc acatggatga gtgatgacga tatccgttta     900

ggctgggcag tgattggttc ccgctcagga agcacgcctc ttttcttttc cagacctgag     960

ggcggaggaa atggggtgcg atttcccgga aaaagtcaaa taggagatcg agggagcgcc    1020

ttatttaaag atcaggcgat cactgcggtc aatcaatttc acaatgtaat ggccgggcag    1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 18
<211> 630
<212> PRT
<213> Artificial Sequence

<220>
<223> mutant amylase

<400> 18
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Ser Asp
    130                 135                 140
Arg Trp Asp Val Thr Gln Asn Ser Leu Glu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190

Ile Glu Leu Pro Asp Asp Gly Asn Tyr Gly Phe Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val
    370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 19
<211> 1896
<212> DNA
<213> Artificial Sequence

<220>
<223> mutant amylase

<400> 19
gctgcaaacg ctgaaactgc aaacaaatcg aatgaggtgg ccgcgtcatc ggtcaaaaac 60

gggaccatcc ttcatgcctg gaattggtcg ttcaatacgt taacacaaaa tatgaaagag 120

attcgtgatg cgggctatgc agccattcag acgtctccga ttaaccaagt aaaggaaggg 180

aaccaaggag ataaaagcat gaggaactgg tactggctgt atcagccgac atcgtaccaa 240

atcggcaacc gttacttagg cactgaacaa gaatttaagg acatgtgtgc agccgcggaa 300

aaatatggcg taaaagtcat tgttgatgcg gttatcaatc ataccaccag cgattacgcc 360

gcgatttctg acgagattaa gcgtattcca aactggaccc atggaaacac acaaattaaa 420

aattgggaag acaggtggga cgtcactcaa aattcattgc ttgggctgta tgattggaat 480

acacagaata ctgaggtgca aacctatctg aaacgtttct tggaaagagc attgaatgac 540

ggagcggacg ggttccgcta tgatgccgcc aagcatatag agcttccgga tgatgggaat 600

tacggcggcc gattttggcc gaatatcaca aatacatcgg cggagttcca atacggagaa 660

atcctgcaag acagcgtttc cagagatact gcttatgcga attatatgaa tgtgacggct 720

tctaactatg ggcattccat cagatccgct ttaaagaacc gtaatctgag tgtgtcgaat 780

atctcccatt atgcatctga cgtgtctgcg gacaagttag tcacatgggt ggaatcacat 840

gatacgtatg ccaatgatga ggaagagtcc acatggatga gtgatgacga tatccgttta 900

ggctgggcag tgattggttc ccgctcagga agcacgcctc ttttcttttc cagacctgag 960

ggcggaggaa atggggtgcg atttcccgga aaaagtcaaa taggagatcg agggagcgcc 1020

ttatttaaag atcaggcgat cactgcggtc aatcaatttc acaatgtaat ggccgggcag 1080

cctgaggaac tctcgaatcc gaatgggaac aatcaaatat tgatgaatca gcgcggctca 1140

aaaggcgttg tgctggctaa tgcgggatcg tcttctgtca ccgtcaatac ttcaacgaaa 1200

ttgcctgacg gcaggtatga taatagggcc ggcgccggtt catttcaagt agcgaacggc 1260

aaactgacag gtacgatcaa tgccagatcc gcggctgttc tttattctga tgatattgga 1320

aatgcgcctc aggtctttct tgagaattac caaacggggg cagtccattc tttcaatgat 1380

cagctgacgg tcaccctgcg tgcaaatgcg aaaacaacaa aagccgttta ccaaatcaat 1440

aatgggcagc agacagcatt taaggatgga gaccgattaa cgatcgggaa aggagatcca 1500

atcggcacga catacaacat caggttaacc ggaacgaacg gcgagggtgc agagagaact 1560

caagaataca cgtttgtcaa aaaagacccg gcccaaacca acatcattgg ctatcaaaat 1620

ccggatcatt ggggccaggt aaatgcttat atttacaaac atgatggagg cagggccata 1680

gaattaaccg gatcgtggcc ggggaaagca atgactaaga atgcaaatgg aatttacacg 1740

ctgacgctgc ctgcgaatgc ggatacggcc aatgccaaag tgatttttaa caatggcagc 1800

gcccaagtgc ccggacagaa ccagcccggc tttgattatg tgcagaatgg tttgtataac 1860

aactccggtt tagacggtta tcttccgcat taataa 1896

<210> 20
<211> 630
<212> PRT
<213> Artificial Sequence

<220>
<223> mutant amylase

<400> 20
Ala Ala Asn Ala Glu Thr Ala Asn Lys Ser Asn Glu Val Ala Ala Ser
1               5                   10                  15
Ser Val Lys Asn Gly Thr Ile Leu His Ala Trp Asn Trp Ser Phe Asn
            20                  25                  30
Thr Leu Thr Gln Asn Met Lys Glu Ile Arg Asp Ala Gly Tyr Ala Ala
        35                  40                  45
Ile Gln Thr Ser Pro Ile Asn Gln Val Lys Glu Gly Asn Gln Gly Asp
    50                  55                  60
Lys Ser Met Arg Asn Trp Tyr Trp Leu Tyr Gln Pro Thr Ser Tyr Gln
65                  70                  75                  80
Ile Gly Asn Arg Tyr Leu Gly Thr Glu Gln Glu Phe Lys Asp Met Cys
                85                  90                  95
Ala Ala Ala Glu Lys Tyr Gly Val Lys Val Ile Val Asp Ala Val Ile
            100                 105                 110
Asn His Thr Thr Ser Asp Tyr Ala Ala Ile Ser Asp Glu Ile Lys Arg
        115                 120                 125
Ile Pro Asn Trp Thr His Gly Asn Thr Gln Ile Lys Asn Trp Glu Asp
    130                 135                 140
Arg Trp Asp Val Thr Gln Asn Ser Leu Leu Gly Leu Tyr Asp Trp Asn
145                 150                 155                 160
Thr Gln Asn Thr Glu Val Gln Thr Tyr Leu Lys Arg Phe Leu Glu Arg
                165                 170                 175
Ala Leu Asn Asp Gly Ala Asp Gly Phe Arg Tyr Asp Ala Ala Lys His
            180                 185                 190
Ile Glu Leu Pro Asp Asp Gly Asn Tyr Gly Gly Arg Phe Trp Pro Asn
        195                 200                 205
Ile Thr Asn Thr Ser Ala Glu Phe Gln Tyr Gly Glu Ile Leu Gln Asp
    210                 215                 220
Ser Val Ser Arg Asp Thr Ala Tyr Ala Asn Tyr Met Asn Val Thr Ala
225                 230                 235                 240
Ser Asn Tyr Gly His Ser Ile Arg Ser Ala Leu Lys Asn Arg Asn Leu
                245                 250                 255
Ser Val Ser Asn Ile Ser His Tyr Ala Ser Asp Val Ser Ala Asp Lys
            260                 265                 270
Leu Val Thr Trp Val Glu Ser His Asp Thr Tyr Ala Asn Asp Glu Glu
        275                 280                 285
Glu Ser Thr Trp Met Ser Asp Asp Asp Ile Arg Leu Gly Trp Ala Val
    290                 295                 300
Ile Gly Ser Arg Ser Gly Ser Thr Pro Leu Phe Phe Ser Arg Pro Glu
305                 310                 315                 320
Gly Gly Gly Asn Gly Val Arg Phe Pro Gly Lys Ser Gln Ile Gly Asp
                325                 330                 335
Arg Gly Ser Ala Leu Phe Lys Asp Gln Ala Ile Thr Ala Val Asn Gln
            340                 345                 350
Phe His Asn Val Met Ala Gly Gln Pro Glu Glu Leu Ser Asn Pro Asn
        355                 360                 365
Gly Asn Asn Gln Ile Leu Met Asn Gln Arg Gly Ser Lys Gly Val Val

370                 375                 380
Leu Ala Asn Ala Gly Ser Ser Ser Val Thr Val Asn Thr Ser Thr Lys
385                 390                 395                 400
Leu Pro Asp Gly Arg Tyr Asp Asn Arg Ala Gly Ala Gly Ser Phe Gln
                405                 410                 415
Val Ala Asn Gly Lys Leu Thr Gly Thr Ile Asn Ala Arg Ser Ala Ala
            420                 425                 430
Val Leu Tyr Ser Asp Asp Ile Gly Asn Ala Pro Gln Val Phe Leu Glu
        435                 440                 445
Asn Tyr Gln Thr Gly Ala Val His Ser Phe Asn Asp Gln Leu Thr Val
    450                 455                 460
Thr Leu Arg Ala Asn Ala Lys Thr Thr Lys Ala Val Tyr Gln Ile Asn
465                 470                 475                 480
Asn Gly Gln Gln Thr Ala Phe Lys Asp Gly Asp Arg Leu Thr Ile Gly
                485                 490                 495
Lys Gly Asp Pro Ile Gly Thr Thr Tyr Asn Ile Arg Leu Thr Gly Thr
            500                 505                 510
Asn Gly Glu Gly Ala Glu Arg Thr Gln Glu Tyr Thr Phe Val Lys Lys
        515                 520                 525
Asp Pro Ala Gln Thr Asn Ile Ile Gly Tyr Gln Asn Pro Asp His Trp
    530                 535                 540
Gly Gln Val Asn Ala Tyr Ile Tyr Lys His Asp Gly Gly Arg Ala Ile
545                 550                 555                 560
Glu Leu Thr Gly Ser Trp Pro Gly Lys Ala Met Thr Lys Asn Ala Asn
                565                 570                 575
Gly Ile Tyr Thr Leu Thr Leu Pro Ala Asn Ala Asp Thr Ala Asn Ala
            580                 585                 590
Lys Val Ile Phe Asn Asn Gly Ser Ala Gln Val Pro Gly Gln Asn Gln
        595                 600                 605
Pro Gly Phe Asp Tyr Val Gln Asn Gly Leu Tyr Asn Asn Ser Gly Leu
    610                 615                 620
Asp Gly Tyr Leu Pro His
625                 630

<210> 21
<211> 8068
<212> DNA
<213> Artificial Sequence

<220>
<223> transformation vector

<400> 21
gggctctgcg tctccaagct ggggcagccc gcctaatgag cgggcttttt tcacgtcacg      60

cgtccatgga gatctttgtc tgcaactgaa aagtttatac cttacctgga acaaatggtt     120

gaaacatacg aggctaatat cggcttatta ggaatagtcc ctgtactaat aaaatcaggt     180

ggatcagttg atcagtatat tttggacgaa gctcggaaag aatttggaga tgacttgctt     240

aattccacaa ttaaattaag ggaaagaata aagcgatttg atgttcaagg aatcacggaa     300

gaagatactc atgataaaga agctctaaaa ctattcaata accttacaat ggaattgatc     360

gaaagggtgg aaggttaatg gtacgaaaat taggggatct acctagaaag ccacaaggcg     420

ataggtcaag cttaaagaac ccttacatgg atcttacaga ttctgaaagt aaagaaacaa     480

cagaggttaa acaaacagaa ccaaaaagaa aaaaagcatt gttgaaaaca atgaaagttg     540

atgtttcaat ccataataag attaaatcgc tgcacgaaat tctggcagca tccgaaggga     600

attcatatta cttagaggat actattgaga gagctattga taagatggtt gagacattac 660

ctgagagcca aaaaactttt tatgaatatg aattaaaaaa aagaaccaac aaaggctgag 720

acagactcca aacgagtctg tttttttaaa aaaaatatta ggagcattga atatatatta 780

gagaattaag aaagacatgg gaataaaaat attttaaatc cagtaaaaat atgataagat 840

tatttcagaa tatgaagaac tctgtttgtt tttgatgaaa aaacaaacaa aaaaaatcca 900

cctaacggaa tctcaattta actaacagcg gccaaactga gaagttaaat ttgagaaggg 960

gaaaaggcgg atttatactt gtatttaact atctccattt taacatttta ttaaacccca 1020

tacaagtgaa aatcctcttt tacactgttc ctttaggtga tcgcggaggg acattatgag 1080

tgaagtaaac ctaaaaggaa atacagatga attagtgtat tatcgacagc aaaccactgg 1140

aaataaaatc gccaggaaga gaatcaaaaa agggaaagaa gaagtttatt atgttgctga 1200

aacggaagag aagatatgga cagaagagca aataaaaaac ttttctttag acaaatttgg 1260

tacgcatata ccttacatag aaggtcatta tacaatctta aataattact tctttgattt 1320

ttggggctat tttttaggtg ctgaaggaat tgcgctctat gctcacctaa ctcgttatgc 1380

atacggcagc aaagactttt gctttcctag tctacaaaca atcgctaaaa aaatggacaa 1440

gactcctgtt acagttagag gctacttgaa actgcttgaa aggtacggtt ttatttggaa 1500

ggtaaacgtc cgtaataaaa ccaaggataa cacagaggaa tccccgattt ttaagattag 1560

acgtaaggtt cctttgcttt cagaagaact tttaaatgga aaccctaata ttgaaattcc 1620

agatgacgag gaagcacatg taaagaaggc tttaaaaaag gaaaaagagg gtcttccaaa 1680

ggttttgaaa aaagagcacg atgaatttgt taaaaaaatg atggatgagt cagaaacaat 1740

taatattcca gaggccttac aatatgacac aatgtatgaa gatatactca gtaaaggaga 1800

aattcgaaaa gaaatcaaaa aacaaatacc taatcctaca acatcttttg agagtatatc 1860

aatgacaact gaagaggaaa aagtcgacag tactttaaaa agcgaaatgc aaaatcgtgt 1920

ctctaagcct tcttttgata cctggtttaa aaacactaag atcaaaattg aaaataaaaa 1980

ttgtttatta cttgtaccga gtgaatttgc atttgaatgg attaagaaaa gatatttaga 2040

aacaattaaa acagtccttg aagaagctgg atatgttttc gaaaaaatcg aactaagaaa 2100

agtgcaataa actgctgaag tatttcagca gtttttttta tttagaaata gtgaaaaaaa 2160

tataatcagg gaggtatcaa tatttaatga gtactgattt aaatttattt agactggaat 2220

taataattaa cacgtagact aattaaaatt taatgaggga taaagaggat acaaaaatat 2280

taatttcaat ccctattaaa ttttaacaag ggggggatta aaatttaatt agaggtttat 2340

ccacaagaaa agaccctaat aaaattttta ctagggttat aacactgatt aatttcttaa 2400

tgggggaggg attaaaattt aatgacaaag aaaacaatct tttaagaaaa gcttttaaaa 2460

gataataata aaaagagctt tgcgattaag caaaactctt tactttttca ttgacattat 2520

caaattcatc gatttcaaat tgttgttgta tcataaagtt aattctgttt tgcacaacct 2580

tttcaggaat ataaaacaca tctgaggctt gttttataaa ctcagggtcg ctaaagtcaa 2640

tgtaacgtag catatgatat ggtatagctt ccacccaagt tagcctttct gcttcttctg 2700

aatgtttttc atatacttcc atgggtatct ctaaatgatt ttcctcatgt agcaaggtat 2760

gagcaaaaag tttatggaat tgatagttcc tctctttttc ttcaactttt ttatctaaaa 2820

caaacacttt aacatctgag tcaatgtaag cataagatgt ttttccagtc ataatttcaa 2880

tcccaaatct tttagacaga aattctggac gtaaatcttt tggtgaaaga atttttttat 2940

gtagcaatat atccgataca gcaccttcta aaagcgttgg tgaatagggc attttaccta 3000

tctcctctca ttttgtggaa taaaaatagt catattcgtc catctaccta tcctattatc 3060

gaacagttga actttttaat caaggatcag tccttttttt cattattctt aaactgtgct 3120

cttaacttta acaactcgat ttgtttttcc agatctcgag ggtaactagc ctcgccgatc 3180

ccgcaagagg cccggcagtc aggtggcact tttcggggaa atgtgcgcgg aacccctatt 3240

tgtttatttt tctaaataca ttcaaatatg tatccgctca tgagacaata accctgataa 3300

atgcttcaat aatattgaaa aaggaagagt atgagtattc aacatttccg tgtcgccctt 3360

attccctttt ttgcggcatt ttgccttcct gtttttgctc acccagaaac gctggtgaaa 3420

gtaaaagatg ctgaagatca gttgggtgca cgagtgggtt acatcgaact ggatctcaac 3480

agcggtaaga tccttgagag ttttcgcccc gaagaacgtt ttccaatgat gagcactttt 3540

aaagttctgc tatgtggcgc ggtattatcc cgtattgacg ccgggcaaga gcaactcggt 3600

cgccgcatac actattctca gaatgacttg gttgagtact caccagtcac agaaaagcat 3660

cttacggatg gcatgacagt aagagaatta tgcagtgctg ccataaccat gagtgataac 3720

actgcggcca acttacttct gacaacgatc ggaggaccga aggagctaac cgcttttttg 3780

cacaacatgg gggatcatgt aactcgcctt gatcgttggg aaccggagct gaatgaagcc 3840

ataccaaacg acgagcgtga caccacgatg cctgtagcaa tggcaacaac gttgcgcaaa 3900

ctattaactg gcgaactact tactctagct tcccggcaac aattaataga ctggatggag 3960

gcggataaag ttgcaggacc acttctgcgc tcggcccttc cggctggctg gtttattgct 4020

gataaatctg gagccggtga gcgtgggtct cgcggtatca ttgcagcact ggggccagat 4080

ggtaagccct cccgtatcgt agttatctac acgacgggga gtcaggcaac tatggatgaa 4140

cgaaatagac agatcgctga gataggtgcc tcactgatta agcattggta actgtcagac 4200

caagtttact catatatact ttagattgat ttaaaacttc atttttaatt taaaaggatc 4260

taggtgaaga tcctttttga taatctcatg accaaaatcc cttaacgtga gttttcgttc 4320

cactgagcgt cagaccccgt agaaaagatc aaaggatctt cttgagatcc tttttttctg 4380

cgcgtaatct gctgcttgca aacaaaaaaa ccaccgctac cagcggtggt ttgtttgccg 4440

gatcaagagc taccaactct ttttccgaag gtaactggct tcagcagagc gcagatacca 4500

aatactgtcc ttctagtgta gccgtagtta ggccaccact tcaagaactc tgtagcaccg 4560

cctacatacc tcgctctgct aatcctgtta ccagtggctg ctgccagtgg cgataagtcg 4620

tgtcttaccg ggttggactc aagacgatag ttaccggata aggcgcagcg gtcgggctga 4680

acggggggtt cgtgcacaca gcccagcttg gagcgaacga cctacaccga actgagatac 4740

ctacagcgtg agctatgaga aagcgccacg cttcccgaag ggagaaaggc ggacaggtat 4800

ccggtaagcg gcagggtcgg aacaggagag cgcacgaggg agcttccagg gggaaacgcc 4860

tggtatcttt atagtcctgt cgggtttcgc cacctctgac ttgagcgtcg atttttgtga 4920

tgctcgtcag gggggcggag cctatggaaa aacgccagca acgcggcctt tttacggttc 4980

ctggcctttt gctggccttt tgctcacatg ttctttcctg cgttatcccc tgattctgtg 5040

gataaccgta ttaccgcctt tgagtgagct gataccgctc gccgcagccg aacgaccgag 5100

cgcagcgagt cagtgagcga ggaagcggaa gagcgcccaa tacgcatgct taagttattg 5160

gtatgactgg ttttaagcgc aaaaaaagtt gctttttcgt acctattaat gtatcgtttt 5220

agaaaaccga ctgtaaaaag tacagtcggc attatctcat attataaaag ccagtcatta 5280

ggcctatctg acaattcctg aatagagttc ataaacaatc ctgcatgata accatcacaa 5340

acagaatgat gtacctgtaa agatagcggt aaatatattg aattaccttt attaatgaat 5400

tttcctgctg taataatggg tagaaggtaa ttactattat tattgatatt taagttaaac 5460

ccagtaaatg aagtccatgg aataatagaa agagaaaaag cattttcagg tataggtgtt 5520

ttgggaaaca atttccccga accattatat ttctctacat cagaaaggta taaatcataa 5580

aactctttga agtcattctt tacaggagtc caaataccag agaatgtttt agatacacca 5640

tcaaaaattg tataaagtgg ctctaactta tcccaataac ctaactctcc gtcgctattg 5700

taaccagttc taaaagctgt atttgagttt atcacccttg tcactaagaa aataaatgca 5760

gggtaaaatt tatatccttc ttgttttatg tttcggtata aaacactaat atcaatttct 5820

gtggttatac taaaagtcgt ttgttggttc aaataatgat taaatatctc ttttctcttc 5880

caattgtcta aatcaatttt attaaagttc atttgatatg cctcctaaat ttttatctaa 5940

agtgaattta ggaggcttac ttgtctgctt tcttcattag aatcaatcct tttttaaaag 6000

tcaatattac tgtaacataa atatatattt taaaaatatc ccactttatc caattttcgt 6060

ttgttgaact aatgggtgct ttagttgaag aataaagacc acattaaaaa atgtggtctt 6120

ttgtgttttt ttaaaggatt tgagcgtagc gaaaaatcct tttctttctt atcttgataa 6180

taagggtaac tattgccgat cgtccattcc gacagcatcg ccagtcacta tggcgtgctg 6240

ctagcgccat tcgccattca ggctgcgcaa ctgttgggaa gggcgatcgg tgcgggcctc 6300

ttcgctatta cgccagctgg cgaaaggggg atgtgctgca aggcgattaa gttgggtaac 6360

gccagggttt tcccagtcac gacgttgtaa aacgacggcc agtgaattcg agctcaggcc 6420

ttaactcaca ttaattgcgt tgcgctcact gcccgctttc cagtcgggaa acctgtcgtg 6480

ccagctgcat taatgaatcg gccaacgcgc ggggagaggc ggtttgcgta ttgggcgcca 6540

gggtggtttt tcttttcacc agtgagacgg gcaacagctg attgcccttc accgcctggc 6600

cctgagagag ttgcagcaag cggtccacgc tggtttgccc cagcaggcga aaatcctgtt 6660

tgatggtggt tgacggcggg atataacatg agctgtcttc ggtatcgtcg tatcccacta 6720

ccgagatatc cgcaccaacg cgcagcccgg actcggtaat ggcgcgcatt gcgcccagcg 6780

ccatctgatc gttggcaacc agcatcgcag tgggaacgat gccctcattc agcatttgca 6840

tggtttgttg aaaaccggac atggcactcc agtcgccttc ccgttccgct atcggctgaa 6900

tttgattgcg agtgagatat ttatgccagc cagccagacg cagacgcgcc gagacagaac 6960

ttaatgggcc cgctaacagc gcgatttgct ggtgacccaa tgcgaccaga tgctccacgc 7020

ccagtcgcgt accgtcttca tgggagaaaa taatactgtt gatgggtgtc tggtcagaga 7080

catcaagaaa taacgccgga acattagtgc aggcagcttc cacagcaatg gcatcctggt 7140

catccagcgg atagttaatg atcagcccac tgacgcgttg cgcgagaaga ttgtgcaccg 7200

ccgctttaca ggcttcgacg ccgcttcgtt ctaccatcga caccaccacg ctggcaccca 7260

gttgatcggc gcgagattta atcgccgcga caatttgcga cggcgcgtgc agggccagac 7320

tggaggtggc aacgccaatc agcaacgact gtttgcccgc cagttgttgt gccacgcggt 7380

tgggaatgta attcagctcc gccatcgccg cttccacttt ttcccgcgtt ttcgcagaaa 7440

cgtggctggc ctggttcacc acgcgggaaa cggtctgata agagacaccg gcatactctg 7500

cgacatcgta taacgttact ggtttcatca aaatcgtctc cctccgtttg aatatttgat 7560

tgatcgtaac cagatgaagc actctttcca ctatccctac agtgttatgg cttgaacaat 7620

cacgaaacaa taattggtac gtacgatctt tcagccgact caaacatcaa atcttacaaa 7680

tgtagtcttt gaaagtatta catatgtaag atttaaatgc aaccgttttt tcggaaggaa 7740

atgatgacct cgtttccacc ggaattagct tggtaccagc tattgtaaca taatcggtac 7800

gggggtgaaa aagctaacgg aaaagggagc ggaaaagaat gatgtaagcg tgaaaaattt 7860

tttatcttat cacttgaaat tggaagggag attctttatt ataagaattg tggaattgtg 7920

agcggataac aattcccaat taaaggagga aggatcaatg attcaaaaac gaaagcggac 7980

agtttcgttc agacttgtgc ttatgtgcac gctgttattt gtcagtttgc cgattacaaa 8040

aacatcagcc gttagcgcag ggagcccc 8068